# EUROPEAN PATENT APPLICATION

(11) **EP 4 523 730 A1**
(43) Date of publication of application: **19.03.2025**
(21) Application number: 23803885.5
(22) Date of filing: 12.05.2023
(51) Int. Cl.: A61N 1/04, A61N 1/36, A61N 2/00

(54) **BRAIN-STIMULATION-DEVICE GUIDE DEVICE, AND APPARATUS AND METHOD FOR MANUFACTURING BRAIN-STIMULATION-DEVICE GUIDE DEVICE**

(30) Priority: 13.05.2022 US 202263342062 P
(71) Applicant: Anymedi Inc., Seoul 05510 (KR)
(72) Inventor: KIM, Guk Bae, Seoul 05510 (KR); KIM, Jong Chan, Seoul 05510 (KR); LIM, Min Je, Seoul 05510 (KR)
(74) Representative: Murgitroyd & Company
(86) International application number: PCT/KR2023/006477
(87) International publication number: WO 2023/219462

(57) **Abstract**

This brain stimulation guide device for positioning a brain stimulation device that stimulates the patient's brain with an electric field or magnetic field, comprises: a face mask mounted on the patient's face; band straps for fixing the face mask to the patient's face; and a procedure device holder which is fixed to the face mask and to which a brain stimulation device having a built-in magnetic coil for generating a magnetic field is mounted, wherein the face mask covers at least portions of the patient's nose, temples, and cheekbones. The brain stimulation guide device can place a brain stimulation device, which is a procedure tool, in an accurate position and thus improve the effect of a procedure.

## Description

### TECHNICAL FIELD

The present disclosure relates to a brain stimulation apparatus guide device that is mounted on the head of a patient and maintains a procedure tool disposed toward a target point of the brain of the patient and an apparatus and method for manufacturing the brain stimulation apparatus guide device.

### BACKGROUND ART

Transcranial magnetic stimulation (TMS) is a non-invasive treatment method for the nervous system, which has the advantage of treating neurological disorders without the need for medication or invasive treatment. TMS uses a change in magnetic field to apply electrical stimulation to a subject, with treatments lasting 20 to 40 minutes. TMS has been shown to be effective in treating neurological and psychiatric disorders such as Parkinson's disease, Alzheimer's disease, cognitive dysfunction, post-traumatic stress disorder, depression, obsessive compulsive disorder, anxiety, autism, headaches, tinnitus, and sleep disorders.

Transcranial direct current stimulation (tDCS) is a non-invasive method that directly stimulates the brain by energizing the subject's primary sensory area with a predetermined intensity of direct current electricity for about 5 minutes to activate neurons of brain cells (to change, i.e. accelerate or suppress, excitability), thereby revealing the neurophysiological function of each area of the brain or restoring the damaged function caused by brain diseases. The treatment time is about 5 to 10 minutes.

Treatment using a non-invasive magnetic or electric field, such as TMS or tDCS, is performed by applying electrical stimulation to a clinically or empirically known stimulation point or by a practitioner moving the stimulation point in small increments to determine the stimulation point.

A conventional brain stimulation apparatus guide device has a problem that the position of the brain area varies slightly from patient to patient and it is difficult to distinguish the lesion point or the exact target point, which reduces the treatment effect.

To extract a more accurate target point, an image obtained by magnetic resonance imaging (MRI) may be used to set the target position. However, even if the point of the brain to be stimulated is specified in the image, it is difficult to confirm the exact position or direction on the actual patient's head, and even if the point to be in contact with a brain stimulation apparatus is specified, if a probe of the stimulation apparatus and the head slightly deviate from each other, stimulation is applied to a point different from the target point.

### DISCLOSURE

### TECHNICAL TASK

The present disclosure has been made in view of the above problems, and it is an object of the present disclosure to provide a brain stimulation apparatus guide device that is customized to fit the position characteristics of the brain of each patient to be stimulated and is capable of accurately guiding a procedure tool to the target point area, thereby enabling a more accurate and safe procedure, and an apparatus and method for manufacturing the brain stimulation apparatus guide device.

### TECHNICAL SOLUTIONS

The present disclosure provides a brain stimulation apparatus guide device for positioning a brain stimulation apparatus for stimulating a patient's brain with an electric field or a magnetic field, the brain stimulation apparatus guide device including a face mask mounted to a face of the patient so as to cover at least a part of a nose, a temple, and a cheek of the patient and a stimulation apparatus holder fixed to the face mask, the stimulation apparatus holder being configured to allow a brain stimulation apparatus having a magnetic coil configured to generate a magnetic field mounted therein to rest thereon, wherein the face mask includes at least one of a first horizontal guide configured to wrap around left and right sides of a nasal bone of the patient, a second horizontal guide located on left and right sides of the temple of the patient, and a third horizontal guide located on the left and right sides of the cheek of the patient.

The face mask may include at least one of a first vertical guide located at an upper part of a nasal bone of the patient, a second vertical guide configured to wrap around a brow bone of the patient, and a third vertical guide configured to cover an upper part of the cheek of the patient. The face mask may further include a fourth vertical guide configured to wrap around a part of a nose tip of the face.

The brain stimulation apparatus may include a transcranial magnetic stimulation (TMS) apparatus or a transcranial direct current stimulation (tDCS) apparatus.

The stimulation apparatus holder may include a resting face configured to wrap around a periphery of a front surface of the brain stimulation apparatus through which the magnetic field is radiated, the resting face comprising an opening formed in the center thereof, a sidewall portion extending from the resting face, the sidewall portion being configured to wrap around a periphery of a side surface of the brain stimulation apparatus, and a fixing protrusion extending from an end of the sidewall portion, the fixing protrusion being configured to allow a part of a rear surface of the brain stimulation apparatus to be hooked thereon.

The stimulation apparatus holder may extend in one direction, and the fixing protrusion may be located on one side of the stimulation apparatus holder in a longitudinal direction.

The brain stimulation apparatus guide device may further include a handle resting portion formed by omitting a part of the sidewall portion, the handle resting portion protruding in a lateral direction, the handle resting portion being configured to allow a handle of the brain stimulation apparatus to be located thereon.

The face mask may further include a band strap configured to fix the face mask to the face of the patient.

The brain stimulation apparatus guide device may include a bridge configured to connect the face mask and the stimulation apparatus holder to each other, wherein the bridge may extend from an upper part of the face mask.

The bridge may include a first bridge connected to the face mask, a second bridge connected to the stimulation apparatus holder, and a fastener configured to fasten the first bridge and the second bridge to each other.

When the position of the stimulation apparatus holder and the position of the face mask at least partially overlap each other, at least a part of an overlap region between the face mask and the stimulation apparatus holder may be omitted.

The face mask may include a strap loop configured to allow the band strap to be fixed thereto, and the strap loop may be provided in at least three on left and right sides and on an upper part of the face mask.

In another aspect, the present disclosure provides a method of manufacturing a brain stimulation apparatus guide device, the method including modeling a face mask configured to cover at least a part of a nose, a temple, and a cheek of a patient based on a three-dimensional image of a face of the patient, a patient's brain map generation step of modeling a three-dimensional image of a brain of the patient, extracting a target point based on the patient's brain map, calculating a procedure position of a brain stimulation apparatus that generates a magnetic field such that the maximum point of a magnetic vector potential of the brain stimulation apparatus overlaps the target point, modeling a first bridge extending from the face mask, and a procedure guide forming step of outputting a procedure guide based on modeling of the face mask and modeling of the bridge, wherein the first bridge includes a fastening portion fastened to a second bridge extending from a ready-made stimulation apparatus holder to which the brain stimulation apparatus is mounted such that the stimulation apparatus holder is located at the procedure position.

The step of modeling the face mask may include at least one of modeling a first horizontal guide configured to wrap around left and right sides of a nasal bone of the face based on the three-dimensional image of the face of the patient, modeling a second horizontal guide located on left and right sides of the temple of the face, and modeling a third horizontal guide located on the left and right sides of the cheek of the face.

The step of modeling the face mask may include at least one of modeling a first vertical guide located at an upper part of a nasal bone of the face based on the three-dimensional image of the face of the patient, modeling a second vertical guide configured to wrap around a brow bone of the face, and modeling a third vertical guide configured to cover an upper part of the cheek of the face.

The step of modeling the face mask may further include modeling a fourth vertical guide configured to wrap around a part of a nose tip of the face.

The step of extracting the target point may include extracting a patient-customized target point to be stimulated with the brain stimulation apparatus based on the patient's brain map and standardized brain stimulation target information.

In another aspect, the present disclosure provides a method of manufacturing a brain stimulation apparatus guide device, the method including modeling a face mask configured to cover at least a part of a nose, a temple, and a cheek of a patient based on a three-dimensional image of a face of the patient, a patient's brain map generation step of modeling a three-dimensional image of a brain of the patient, extracting a target point from the patient's brain map, calculating a procedure position of a brain stimulation apparatus that generates a magnetic field from a three-dimensional image of a head of the patient such that the maximum point of a magnetic vector potential of the brain stimulation apparatus overlaps the target point, modeling a stimulation apparatus holder taking into account the shape of the brain stimulation apparatus and the procedure position, and a brain stimulation apparatus guide device forming step of outputting a brain stimulation apparatus guide device based on modeling of the face mask and modeling of the stimulation apparatus holder.

The step of modeling the face mask may include at least one of modeling a first horizontal guide configured to wrap around left and right sides of a nasal bone of the face based on the three-dimensional image of the face of the patient, modeling a second horizontal guide located on left and right sides of the temple of the face, and modeling a third horizontal guide located on the left and right sides of the cheek of the face.

The step of modeling the face mask may include at least one of modeling a first vertical guide located at an upper part of a nasal bone of the face based on the three-dimensional image of the face of the patient, modeling a second vertical guide configured to wrap around a brow bone of the face, and modeling a third vertical guide configured to cover an upper part of the cheek of the face. The step of modeling the face mask may further include modeling a fourth vertical guide configured to wrap around a part of a nose tip of the face.

The method may further include modeling a bridge configured to connect the face mask and the stimulation apparatus holder to each other when the face mask and the stimulation apparatus holder are spaced apart from each other, wherein the brain stimulation apparatus guide device forming step may include outputting the brain stimulation apparatus guide device including the bridge.

When the position of the stimulation apparatus holder and the position of the face mask at least partially overlap each other,

the step of modeling the face mask may include modeling the face mask with at least a part of the face mask that overlaps the stimulation apparatus holder omitted.

The step of extracting the target point may include extracting a patient-customized target point to be stimulated with the brain stimulation apparatus based on the patient's brain map and standardized brain stimulation target information.

In a further aspect, the present disclosure provides an apparatus for manufacturing a brain stimulation apparatus guide device, the apparatus including a face modeling module configured to generate a three-dimensional image of a face of a patient based on an image of a head of the patient, a brain map generation module configured to model a three-dimensional image of a brain of the patient based on an MRI image of the brain of the patient,

a target point determination module configured to extract a target point from the generated brain map, a procedure position determination module configured to determine a procedure position of a brain stimulation apparatus that generates a magnetic field such that the maximum point of a magnetic vector potential of the brain stimulation apparatus is located at the target point, a guide device modeling module configured to model a face mask configured to cover at least a part of a nose, a temple, and a cheek of the patient and a stimulation apparatus holder configured to mount the brain stimulation apparatus at the procedure position based on the three-dimensional image of the face of the patient, and a guide device forming module configured to output a brain stimulation apparatus guide device based on modeling of the face mask and the stimulation apparatus holder.

The guide device modeling module may model a bridge configured to connect the face mask and the stimulation apparatus holder to each other when the face mask and the stimulation apparatus holder are spaced apart from each other, and the guide device forming module may output the brain stimulation apparatus guide device including the bridge.

### ADVANTAGEOUS EFFECTS

According to at least one embodiment of the present disclosure, it is possible to dispose a brain stimulation apparatus, which is a procedure tool, at a correct position, thereby improving the effectiveness of the procedure.

A brain stimulation position does not change even if a patient moves, reducing the cumbersomeness of having to determine a procedure position each time during repeated procedures.

Further scope of applicability of the present disclosure will become apparent from the following detailed description. However, various changes and modifications within the idea and scope of the present disclosure will be apparent to those skilled in the art, and therefore the detailed description and specific embodiments, such as preferred embodiments of the present disclosure, should be understood to be given by way of example only.

### DESCRIPTION OF DRAWINGS

FIGs. 1 and 2 are perspective views showing an example of a brain stimulation apparatus guide device according to an aspect of the present disclosure in different directions.
FIG. 3 is a perspective view showing a face mask and band strap of the brain stimulation apparatus guide device according to the aspect of the present disclosure.
FIGs. 4 and 5 are perspective views showing a wearing state of the brain stimulation apparatus guide device according to the aspect of the present disclosure in different directions.
FIGs. 6 and 7 are views showing a bridge fastening mode of the brain stimulation apparatus guide device according to the aspect of the present disclosure.
FIG. 8 is a view showing another embodiment of the brain stimulation apparatus guide device according to the aspect of the present disclosure.
FIG. 9 is a view showing a further embodiment of the brain stimulation apparatus guide device according to the aspect of the present disclosure.
FIG. 10 is a view showing an apparatus for manufacturing a brain stimulation apparatus guide device according to another aspect of the present disclosure.
FIG. 11 is a view showing a method of manufacturing a brain stimulation apparatus guide device according to a further aspect of the present disclosure.
FIGs. 12A to 13C are views showing a manufacturing process according to the method of manufacturing the brain stimulation apparatus guide device according to the further aspect of the present disclosure.

### BEST MODE FOR DISCLOSURE

Hereinafter, preferred embodiments of the present disclosure will be described in detail with reference to the accompanying drawings. The advantages and features of the present disclosure, and methods of achieving them, will become apparent upon reference to the embodiments described in detail in conjunction with the accompanying drawings. The present disclosure, however, is not limited to the embodiments disclosed herein, but may be embodied in many different forms, and these embodiments are provided merely to make the disclosure complete and to give those of ordinary skill in the art to which the present disclosure belongs a complete idea of the scope of the present disclosure, which is defined by the scope of the claims. Throughout the specification, like reference numerals refer to like components.

Unless otherwise defined, all terms (including technical and scientific terms) used in this specification have the same meanings as those commonly understood by a person having ordinary skill in the art to which the present disclosure pertains. It will be further understood that terms, such as those defined in commonly used dictionaries, should be interpreted as having meanings consistent with their meanings in the context of the relevant art and the present disclosure, and are not to be interpreted in an idealized or overly formal sense unless expressly so defined herein.

The terms used in this specification are provided only to explain the embodiments, but are not intended to restrict the present disclosure. A singular representation may include a plural representation unless it represents a definitely different meaning from the context. The term "comprises" and/or "comprising" as used herein does not exclude the presence or addition of one or more other components in addition to those mentioned.

As used herein, "computer" includes a variety of apparatuses capable of performing computational processing and visually presenting the results to a user. For example, the computer may be not only a desktop computer or a laptop computer but also a smartphone, a tablet computer, a cellular phone, a personal communication service phone (PCS phone), a synchronous or asynchronous International Mobile Telecommunication-2000 (IMT-2000) mobile terminal, a palm personal computer, or a personal digital assistant (PDA). The computer may also be a medical device for acquiring or observing angiographic images.

As used herein, "medical image data" means image data acquired by a medical imaging device (e.g., a computed tomography (CT) device or a magnetic resonance imaging (MRI) device).

As used herein, "target point" means a specific point in the brain associated with a symptom. The magnetic field generated by a TMS probe must be targeted to the point where the vector potential of the magnetic field is maximized in order to provide effective treatment. The treatment effect may be improved by positioning a brain stimulation apparatus such that the point where the electrical stimulation of tDCS is maximally concentrated coincides with the target point.

Hereinafter, a customized surgical guide, a method of generating a customized surgical guide, and a program according to embodiments of the present disclosure will be described in detail.

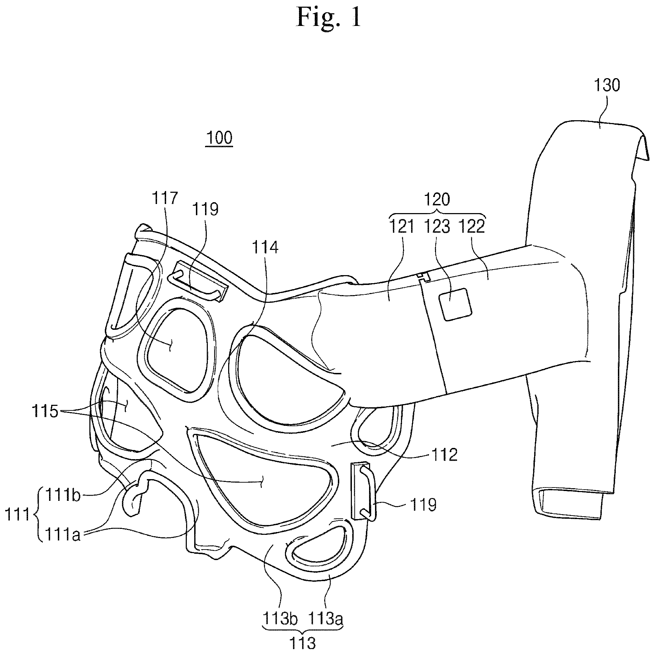

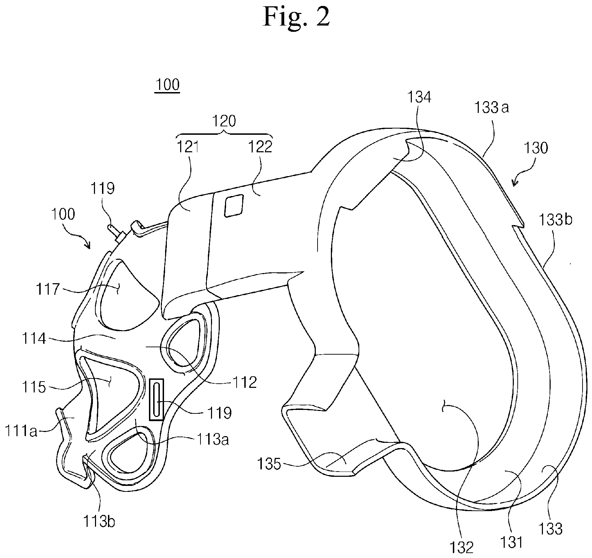

FIGs. 1 and 2 are perspective views showing an example of a brain stimulation apparatus guide device 100 according to an aspect of the present disclosure in different directions.

The brain stimulation apparatus guide device 100 of the present disclosure is a brain stimulation apparatus guide device 100 for fixing a non-invasive brain stimulation apparatus, such as TMS or tDCS, which provides electrical stimulation to a target point in the brain of a user to the head of the user.

Transcranial magnetic stimulation (TMS) is a non-invasive treatment method for the nervous system, which has the advantage of treating neurological disorders without the need for medication or invasive treatment. TMS uses a change in magnetic field to apply electrical stimulation to a subject, with treatments lasting 20 to 40 minutes. TMS has been shown to be effective in treating neurological and psychiatric disorders such as Parkinson's disease, Alzheimer's disease, cognitive dysfunction, post-traumatic stress disorder, depression, obsessive compulsive disorder, anxiety, autism, headaches, tinnitus, and sleep disorders.

Transcranial direct current stimulation (tDCS) is a non-invasive method that directly stimulates the brain by energizing the subject's primary sensory area with a predetermined intensity of direct current electricity for about 5 minutes to activate neurons of brain cells (to change, i.e. accelerate or suppress, excitability), thereby revealing the neurophysiological function of each area of the brain or restoring the damaged function caused by brain diseases. The treatment time is about 5 to 10 minutes.

In a procedure that requires the brain stimulation apparatus to be located at the same position for a predetermined period of time, the brain stimulation apparatus may be held at the procedure position by a practitioner, or the brain stimulation apparatus may be fixed at the procedure position using a stand.

It is difficult for the practitioner to hold the brain stimulation apparatus at the same position for tens of minutes, and even when the stand is utilized, the stimulation point may be off target if a patient moves.

In addition, the practitioner must determine the procedure position during the procedure such that the target point and the stimulation point match, which may be inaccurate depending on the practitioner's skill, and even if referring to MRI images, the direction of the brain stimulation apparatus may be misaligned depending on the shape of the actual head, making it difficult to align the brain stimulation apparatus at the correct procedure position.

In particular, an electromagnetic stimulation apparatus, such as TMS, is ineffective if the magnetic stimulation does not reach the correct point sufficiently, and the effectiveness plummets when the probe is moved away from the scalp by only a few millimeters.

To address the above problems, the present disclosure provides a brain stimulation apparatus guide 100 that can be fixed to the head of a user. A brain stimulation apparatus 10 may be fixed to the head such that the brain stimulation apparatus 10 is held at the position where the target point and the stimulation point match.

In the medical technology industry, there are various auxiliary devices for the brain stimulation apparatus 10, but due to differences in body structure and shape of each patient, it is difficult to apply a unified medical device, and a practitioner or operator selects or modifies the tool according to the patient, but there is a problem that the accuracy is poor and the effectiveness varies greatly depending on the practitioner.

Recently, additive manufacturing, also known as "3D printing," has been introduced to the medical technology industry to enable personalized medical care. It is also possible to manufacture customized medical devices that are adapted to the shape of the patient's body.

The present disclosure relates to a method of designing a patient-customized brain stimulation apparatus guide device 100 and manufacturing a patient-specific brain stimulation apparatus guide device 100 using such 3D printing technology and to a patient-customized brain stimulation apparatus guide device 100.

The brain stimulation apparatus guide device 100 of the present disclosure may include a face mask 110 that is fixed to the face of a user and a stimulation apparatus holder 130 configured to hold a brain stimulation apparatus 10. The brain stimulation apparatus guide device may include a bridge 120 configured to connect the face mask 110 and the stimulation apparatus holder 130 to each other depending on the position of the stimulation apparatus holder 130.

The face mask 110 is a unit fixed to the face of a patient, and may cover at least a part of the nose, temples, and cheeks of the patient. The face mask 110 may be configured to cover only the upper surface of the face, leaving the part 115 corresponding to the eyes open for ease of use, as covering the lower part of the face may be uncomfortable.

The face mask 110 may include curved surfaces that conform to the curves of the face of the patient. In a human face, a part of the face such as the nose, cheeks, and temples, has bones located adjacent to the skin, and the shape of the bones does not change, and therefore the position of the bones located adjacent to the skin is a good reference (landmark).

Since the face bones, such as nasal bones and cheeks, are more uneven than the skull, making it easier to accurately locate the same, a face mask 110 that fits the face of the patient to conform to the unevenness of the face bones may be included.

To align the position of the brain stimulation apparatus guide device 100, an x-axis, y-axis, and z-axis must be aligned. The x-axis refers to a horizontal direction, the y-axis refers to a vertical direction, and the z-axis refers to a forward-rearward direction.

To guide the position in the horizontal direction, symmetrical parts of the patient's face bones may be used as landmarks based on the patient's face bone geometry. The landmarks may be the parts of the face bones that protrude close to the skin, such as the left and right sides of the nasal bones, the left and right sides of the temples, and the cheekbones.

The forehead area has thin skin but a rounded shape that makes it difficult to accurately measure the position thereof, and therefore a somewhat lateralized area may be a landmark.

The face mask 110 may include a first horizontal guide 111a that wraps around the left and right sides of the patient's nasal bone. The first horizontal guide 111a may be shaped to wrap around the left and right sides of the nasal bone located near the root of the nose.

The patient's temple may be a lateral bend in the forehead and may serve as a reference point for the left and right directions. The face mask 110 may include a second horizontal guide 112 that contacts the temple.

The lateral parts of the cheekbones may not have thick skin and may be in contact with the face mask to fix the position of the face mask 110. Therefore, the face mask may further include a third horizontal guide 113a that contacts both sides of the cheekbone.

The skin at the three positions is thin, such that the face mask 110 does not change in position when pressed, even if there is a lot of flesh on the face, and may be utilized as a guide to fix the face mask 110 in a constant position.

Although only parts of the three horizontal guides 111a, 112, and 113a may be utilized, it is advantageous to include the plurality of horizontal guides 111a, 112, and 113a to stably support the face mask.

For vertical guides 111b, 114, and 113b for guiding the position in the y-axis direction, parts that can support the face mask 110 at the lower side may be referenced, since there is a downwardly pressing force due to gravity.

For example, the front part of the nose bone, the upper surface part of the protrusion of the eyebrow bone, and the upper part of the cheekbone may be the reference position. The face mask 110 of the present disclosure may include a first vertical guide 111b located at an upper part of the nasal bone. The first vertical guide 111b may have a continuous shape with the first horizontal guide 111a and may be the most important guide for setting landmarks on the face mask 110.

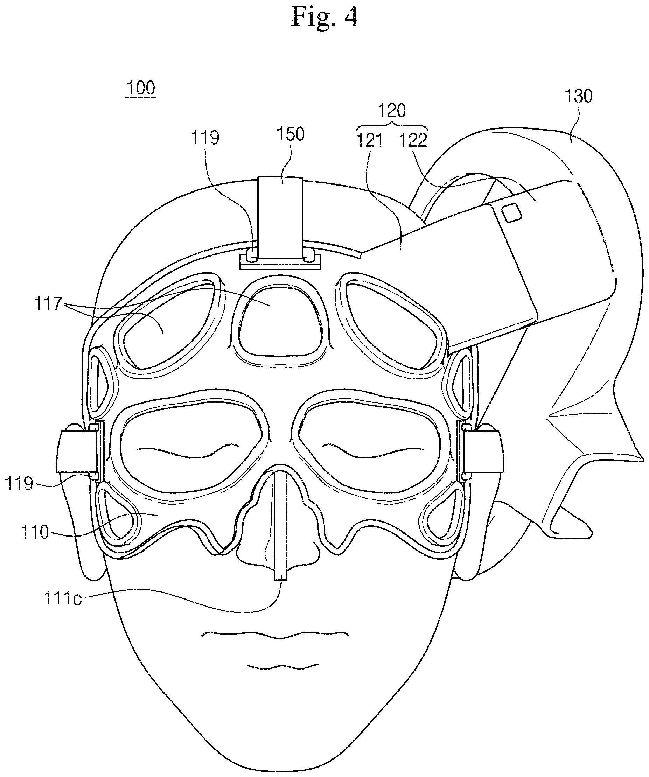

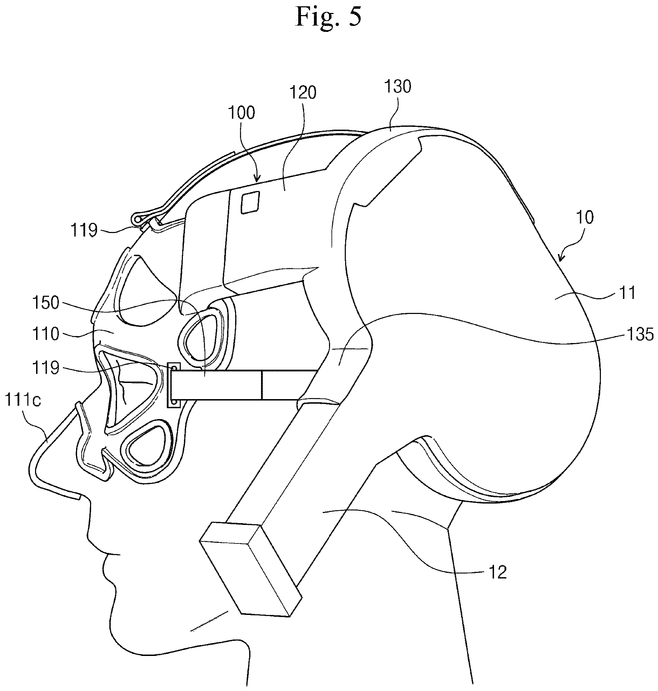

It is possible to implement a face mask 110 that includes the first vertical guide 111b in a shape that covers only the upper part of the nose where the nasal bone is located, as shown in FIGs. 1 and 2, and extends downward to wrap around the nasal bridge or nostrils (see FIGs. 4 and 5).

However, the nasal bridge or nostrils may be composed of cartilage or skin only and may serve as an auxiliary guide of the first vertical guide 111b rather than a main guide for positioning the face mask 110.

The nasal bone may be curved where it meets the forehead bone at the top, thereby constituting the first vertical guide 111b of the face mask 110 in a shape corresponding to a depression in the area between the eyes.

The brow portion of the forehead bone may protrude and may have an inwardly recessed shape relative to the protruding forehead bone at the top of the brow. The face mask 110 may include a second vertical guide 114 that rests on the top of the patient's eyebrows to stabilize the position of the face mask 110 once the face mask 110 rests on the top of the eyebrows.

The cheekbones may also protrude forward to form an upper surface angled upward, and may include a third vertical guide 113b that rests on the upper part of the cheekbones. The vertical guides 111b, 114, and 113b may serve to support the weight of the brain stimulation apparatus guide device 100 and the weight of the brain stimulation apparatus 10, and thus need to have sufficient area.

In addition, the face mask 110 may further include a fourth vertical guide 111c that wraps around a part of the nose tip of the patient's face.

However, the larger the shape of the face mask 110, the heavier the weight of the face mask 110 and the more uncomfortable it may be to use, and therefore the face mask 110 may have an opening 117 with parts other than the vertical guides 111b, 114, and 113b and the horizontal guides 111a, 112, and 113a removed, as shown in FIG. 1.

The opening 117 may provide comfort for wearing the face mask 110 and may reduce the weight of the brain stimulation apparatus guide device 100. The opening may also reduce 3D printing time and reduce manufacturing costs by reducing the amount of printing solution required.

However, if the face mask 110 has a small area, stiffness may be an issue. As shown in FIG. 1, the stiffness of the face mask 110 may be improved by making the thickness of the peripheral portion of the opening portion 117 thicker than the other portions.

In addition to the vertical guides 111b, 114, 113b, and 111c or the horizontal guides 111a, 112, and 113a, the face mask 110 may be hung over the top of the ears to align the position of the face mask and fix the face mask to the face. However, unlike eyeglasses, the weight of the face mask 110 makes hanging it over the ear less supportive, and the cartilaginous nature of the ear limits the ability to achieve accurate vertical alignment.

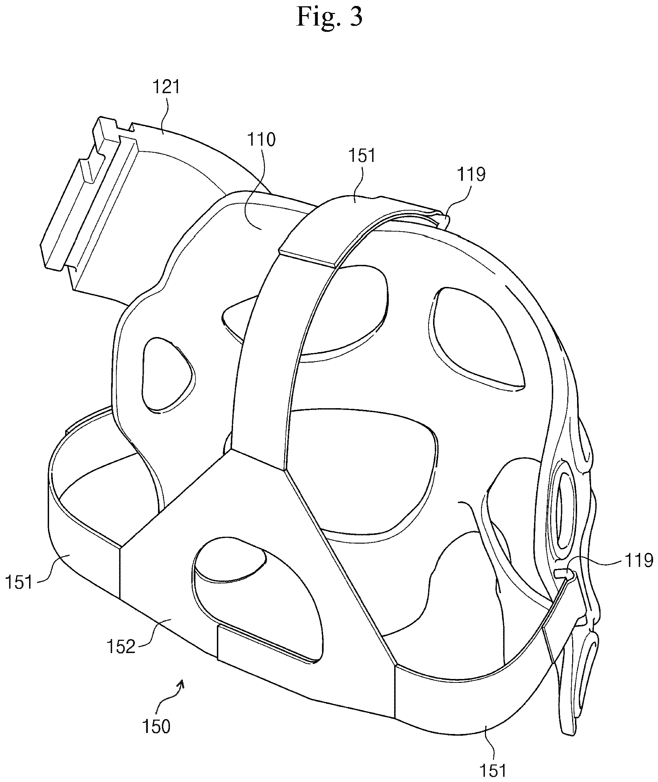

FIG. 3 is a perspective view showing the face mask 100 and a band strap 150 of the brain stimulation apparatus guide device 100 according to the aspect of the present disclosure.

The face mask 110 of the present disclosure may be fixed using the band strap 150 that wraps around the back of the head. The band strap 150 may pull the face mask 110, which is located in front of the patient's face, in a rearward direction to maintain a tight fit on the face and fix the position of the face.

The band strap 150 may be further provided to fix the position of the face mask 110 in the z-axis direction (forward-rearward direction). The face mask 110 may be provided on left and right sides thereof with strap connections 119 to which the band straps 150 can be connected, and the strap connection may be further provided on an upper side thereof.

The band strap 150 may be hooked to the strap connections 119 using hooks at the ends or may be adjustable in length using Velcro. The band strap 150 may include an elastic rubber band.

The band strap 150 may have a straight shape connected from the left and right sides of the face mask 110, and may be further connected from the upper side of the face mask 110 to form a T-shape.

The embodiment shown in FIG. 3 may include a circular band connection 152 that wraps around the occipital region to increase comfort in the T-shape. A strap 151 extends from the connection 152 to each strap connection 119.

FIGs. 4 and 5 are perspective views showing a wearing state of the brain stimulation apparatus guide device 100 according to the aspect of the present disclosure in different directions.

The stimulation apparatus holder 130 may have a shape corresponding to the shape of the brain stimulation apparatus 10. As shown in FIG. 5, the brain stimulation apparatus 10 may include a handle 12 and a probe 11 in which a magnetic field is generated, and the stimulation apparatus holder 130 serves to hold the probe 11 at a procedure position on the patient's head.

In the figure, the probe 11 has an elongated elliptical shape, but is not necessarily limited thereto. The stimulation apparatus holder 130 may be designed so as to correspond to the shape of the brain stimulation apparatus 10 to be used on the patient.

A magnetic field radiation surface of the brain stimulation apparatus 10 may include, for example, a resting portion 131 (FIG. 2) having an opening 132 (FIG. 2) formed to open toward the patient's head and a sidewall portion 133 (FIG. 2) that wraps around the perimeter of the brain stimulation apparatus 10 to fix the brain stimulation apparatus 10.

The brain stimulation apparatus 10 may include a fixing protrusion 134 that protrudes from one side of the sidewall portion 133 and is located at the rear of the brain stimulation apparatus 10 to prevent the brain stimulation apparatus 10 from being dislodged from the stimulation apparatus holder 130. The fixing protrusion 134 may be located at a long end when the stimulation apparatus holder 130 is elongated in one direction to facilitate removal of the brain stimulation apparatus 10.

Excluding the portion where the fixing protrusion 134 is located, the sidewall portion 133 needs not necessarily have a thickness corresponding to the thickness of the brain stimulation apparatus 10, and the height of the sidewall portion 133 may be such that the probe 11 can be received, as shown in FIG. 2, and may be differently (133a, or 133b) configured depending on position.

Because the brain stimulation apparatus 10 is planar and the patient's head has a curved surface, the stimulation apparatus holder 130 may be disposed such that a central part of the stimulation apparatus holder contacts the patient's head and a peripheral part of the stimulation apparatus holder 130 may be spaced apart from the head.

The brain stimulation apparatus 10 may include a probe 11 and a handle 12 for a practitioner to position the brain stimulation apparatus 10 at the patient's head and radiate TMS. In this case, a configuration for fixing the probe 11 and the handle 12 may be provided at the stimulation apparatus holder 130, and particularly a sidewall portion 133 of the stimulation apparatus holder 130 may be opened at one side to allow the handle 12 to pass therethrough to form a handle resting portion 135.

The handle resting portion 135 may support only the part of the brain stimulation apparatus 10 where the handle 12 and the probe 11 are connected to each other, and the remainder of the handle 12 may not be in contact with the brain stimulation apparatus guide device 100. If the brain stimulation apparatus 10 does not include the handle 12, the handle resting portion 135 may be omitted. For example, the brain stimulation apparatus 10 may be a TMS apparatus, and the handle 12 may be a handle portion of a probe of the TMS device.

The target point of the patient's brain may vary depending on the patient's disease. If the target point is at the rear of the brain, the stimulation apparatus holder 130 may include a bridge 120 connecting the stimulation apparatus holder 130 to the face mask 110, as apparatus holder should be located at the back of the head, as shown in FIG. 1.

The bridge 120 is a member that connects the brain stimulation apparatus 10 and the face mask 110 when they are spaced apart from each other, and may have a predetermined thickness for rigidity.

The bridge 120 is coupled to one side of the stimulation apparatus holder 130, and since the brain stimulation apparatus 10 is planar but the patient's head has a curved surface, the perimeter of the stimulation apparatus holder 130 is spaced apart from the head, and therefore the bridge 120 does not extend along the head and may have a shape spaced apart from the head.

Since the stimulation apparatus holder 130 is compatible with the same brain stimulation apparatus 10 regardless of the patient, the stimulation apparatus holder 130 may be provided ready-made for multiple uses. In this case, only the face mask 110 may be designed and made on a patient-specific basis, and the stimulation apparatus holder 130 and the face mask 110 may be detachably coupled to each other.

Since the shape of the head and the target point of brain stimulation are different for each patient, a first bridge 121 and the face mask 110 connected to the ready-made apparatus holder 130 may be designed such that the position or angle of the stimulation apparatus holder can be fixed to the target point in advance during treatment.

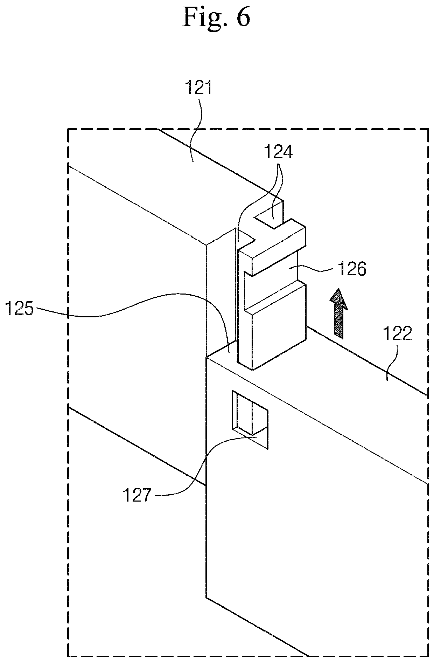

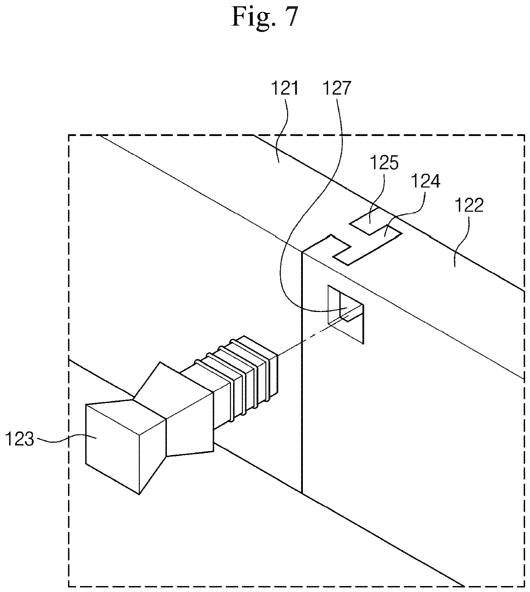

Meanwhile, the first bridge 121 or a second bridge 122 may be detachably coupled to the face mask 110 or the stimulation apparatus holder 130 as needed, although not shown. As shown in FIG. 6 and 7, the bridge 120 may include a first bridge 121 located on the face mask 110 and a second bridge 122 connected to the stimulation apparatus holder 130. The first bridge 121 may have a shape that can be connected to the second bridge 122 such that the stimulation apparatus holder 130, which will be described later, is located at a procedure position.

The first bridge 121 and the second bridge 122 may have irregularities formed at their ends to allow them to slide together in one direction and restrict movement in the other direction. To limit movement in the sliding direction and connect the first bridge 121 and the second bridge 122, a pin-shaped fastener 123 (FIG. 7) extending through the first bridge 121 and the second bridge 122 may be used.

FIGs. 6 and 7 are views showing a fastening mode of the bridge 120 of the brain stimulation apparatus guide device 100 according to the aspect of the present disclosure. The first bridge 121 and the second bridge 122 include fastening rails 124 and 125 extending in one direction for slide engagement.

A convex portion of the second fastening rail 125 of the second bridge 122 is inserted into a concave portion of the first fastening rail 124 of the first bridge 121 and a concave portion of the second fastening rail 125 is engaged with a convex portion of the first fastening rail 124 to enable slide engagement in a width direction of the bridge 120.

Movement of the slide-type fastening rails 124 and 125 in the thickness direction and the longitudinal direction of the bridge 120 may be restricted, but movement thereof in the sliding direction (the direction of the arrow in FIG. 6), i.e., the width direction may not be completely blocked.

In the present disclosure, therefore, a fastening recess 126 may be formed in the first bridge 121, a fastening hole 127 that overlaps the fastening recess 126 of the first bridge 121 may be formed in the second bridge 122, and a fastener 123 may be inserted into the fastening hole 127 to fix the position of the fastening recess 126. The fastener 123 restricts movement of the first bridge 121 and the second bridge 122, and insertion of the fastener 123 into the fastening hole 127 may stably fix the stimulation apparatus holder 130.

The fastener 123 may be locked in the form of a buckle in addition to a pin fastening mode, and the fastening mode of the first bridge 121 and the second bridge 122 is not limited thereto.

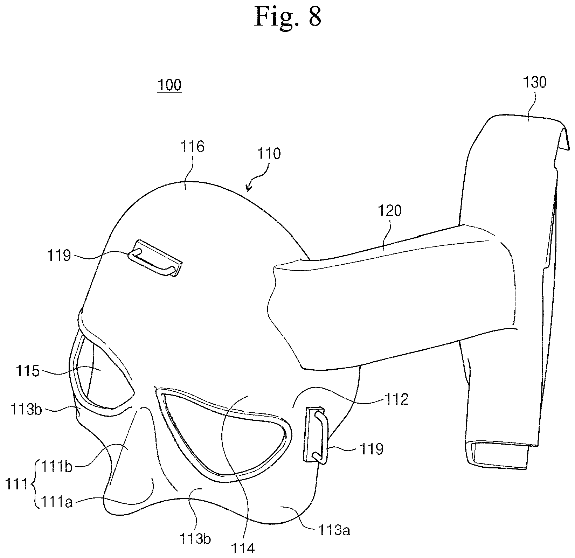

FIG. 8 is a view showing another embodiment of the brain stimulation apparatus guide device 100 according to the aspect of the present disclosure. As in the embodiment shown in FIG. 8, the face mask 110 may be configured as a solid type and may cover a forehead portion 116 to allow the face mask 110 to more stably rest on the face.

The bridge 120 may be configured as an integrated type, rather than a fastened type, to form a single module in which the stimulation apparatus holder 130 and the face mask 110 are connected to each other. This may result in a larger 3D printed piece than the previous embodiment, which may be more expensive and less versatile, but may reduce the design time for coordinating components configured to hold the stimulation apparatus holder in place and may improve stability of the bridge 120.

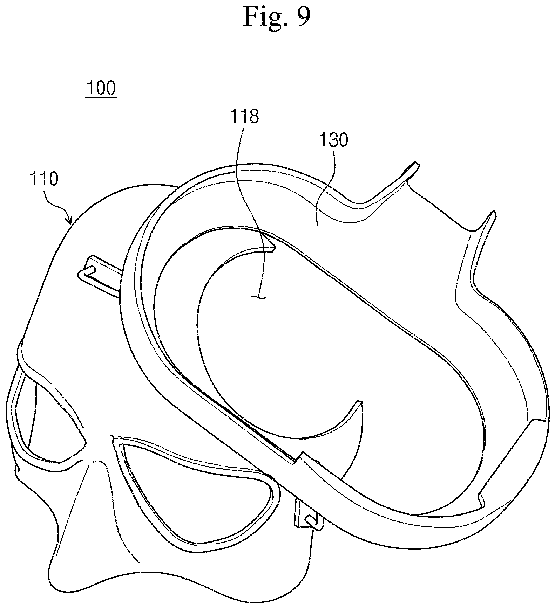

FIG. 9 is a view showing a further embodiment of the brain stimulation apparatus guide device 100 according to the aspect of the present disclosure. The stimulation apparatus holder 130 may overlap the face mask, as shown in FIG. 9, as the brain stimulation apparatus 10 should be located on the forehead if the target position is located in the front of the brain.

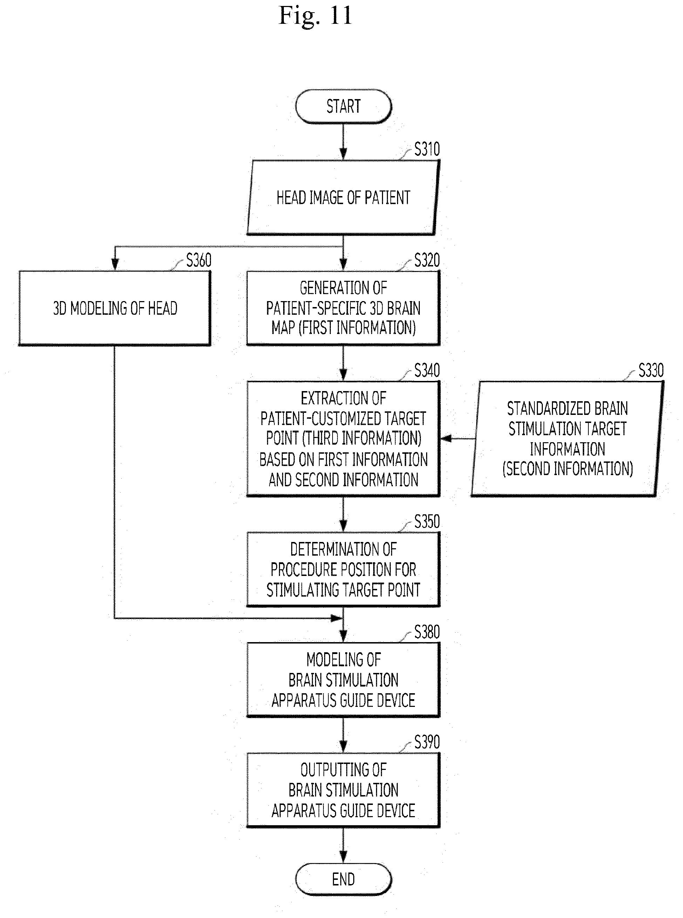

In this case, a part of the face mask 110 may be omitted, and the stimulation apparatus may be manufactured in the form in which the bridge 120 is omitted and the stimulation apparatus holder 130 and the face mask 110 are directly connected to each other. As will be described later in a method of manufacturing the brain stimulation apparatus guide device 100 of FIGs. 11 to 13, the brain stimulation apparatus guide device 100 may be shaped as shown in FIG. 9 with the target point located in the front of the brain in the case of depression.

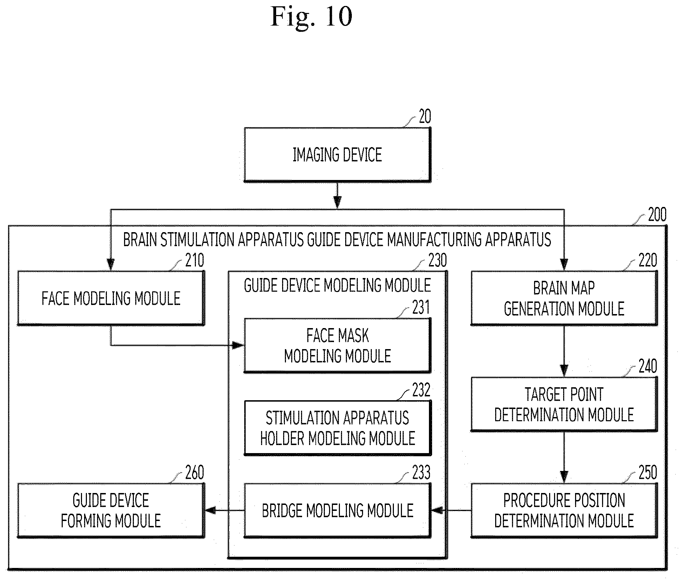

FIG. 10 is a view showing a brain stimulation apparatus guide device manufacturing apparatus 200 according to another aspect of the present disclosure. The brain stimulation apparatus guide device manufacturing apparatus 200 of the present disclosure may include a face modeling module 210, a brain map generation module 220, a guide device modeling module 230, and a guide device forming module 260.

The face modeling module 210, the brain map generation module 220, and the guide device modeling module 230, with the exception of the guide device forming module 260, may be operated integrally and not distinctly as control logic implemented in a single system. Although described separately for ease of description, the operations of the plurality of modules may be performed in a single module without necessarily being limited thereto.

The brain stimulation apparatus guide device manufacturing apparatus 200 may manufacture a patient-customized brain stimulation apparatus guide device 100 using a head image of a patient, and thus may receive image information of the patient from an imaging device 20. The imaging device 20 may include at least one of medical imaging devices, such as a magnetic resonance imaging (MRI) device, a functional MRI device, a computed tomography (CT) device, a positron emission tomography (PET) device, and an optical scanner, which are capable of capturing images of the brain in the head.

Magnetic resonance imaging (MRI) utilizes a magnetic field to capture images of elements that resonate with the magnetic field, and may show various chemical properties (e.g., chemical shift, spin lattice relaxation time, and spin relaxation time), blood flow velocities, diffusion coefficients, and other physical quantities in the human body.

Functional magnetic resonance imaging (functional MRI) is technology that uses a magnetic field to indirectly observe changes in blood flow, particularly changes in the concentration of reduced hemoglobin (deoxyhemoglobin), which delivers oxygen to tissues and takes in carbon dioxide, as neural activity in the brain is activated, and may be used to capture images of changes in the brain when a specific function is performed.

Computed tomography (CT) is a method of measuring the X-ray absorption coefficient of sections of the human body by transmitting X-ray beams in multiple directions and synthesizing captured images by obtaining the CT value of each cross-section using a filter-corrected inverse protrusion method.

Positron emission tomography (PET) images the three-dimensional distribution of certain metabolic activities in vivo.

A brain image may be captured using such a three-dimensional image capturing technique, and a brain map may be generated based thereon. The brain map generation module 220 may receive the brain image of the patient captured using the three-dimensional technique from the imaging device 20 and generate a brain map (first information) of the patient. In some embodiments, a brain region associated with the disease may be further identified in the patient's brain map.

Then, a standardized stimulation point (second information) that requires brain stimulation based on disease symptoms may be matched on the patient's brain map (first information) and extracted as a final target point.

For example, in the case of treating dementia, the target point of the angular gyrus or the left and right lateral parietal lobes may be a standardized position to perform brain stimulation i.e., standardized brain stimulation target information (second information). In this case, in order to locate the part in the brain image of the patient using brain stimulation target information to treat the same symptom, a process of extracting a patient-customized target point (third information) by matching a standardized segmentation map that classifies the human brain into 52 regions, i.e., the standardized brain stimulation target information (second information), and the 3D brain map of the patient to find the target position to perform the brain stimulation may be performed.

In addition, the brain map generation module 220 may obtain additional information that may assist in extracting a final target point by performing a brain stimulation test on the patient using the brain stimulation apparatus or the like in order to extract a patient-specific accurate target point for brain stimulation.

All the target points obtained by matching the standard information on the brain map of the patient may not be the parts that have a therapeutic effect by stimulation of the stimulation apparatus. Therefore, a target point determination module 240 may extract an anomalous point corresponding to the part corresponding to the standardized stimulation point as a target point (third information) based on the standardized stimulation point (second information) and the patient's brain map.

A procedure position determination module 250 determines the position of the brain stimulation apparatus 10 such that the final target point is located within an effective range of the effect of a magnetic field or electric field of the brain stimulation apparatus 10. When the effective range of the brain stimulation apparatus 10 is narrow, the procedure position determination module 250 determines not only the position but also the angle of the brain stimulation apparatus 10.

In order to maximize the effectiveness of the brain stimulation apparatus 10, in the case of TMS, the procedure position may be determined such that the maximum point of the magnetic vector potential of the magnetic field is the target point. In addition to TMS, the point of maximum effectiveness for electromagnetic or ultrasonic stimulation may be set as the target point.

The 3D image of the patient captured by the imaging device may include information about the patient's skin and skeleton as well as the brain. The face modeling module 210 may utilize the information to generate a 3D representation of the face and extract landmark points corresponding to the vertical guides 111b, 114, 113b, and 111c and the horizontal guides 111a, 112, and 113a based thereon.

Since an image of the head (meaning the shape of the entire head, including the head shape and the face shape) can be captured externally and can be captured accurately using inexpensive equipment, 3D scanning may be performed using a separate capturing device to capture the image of the shape of the head separately from the brain image, and face modeling may be performed. However, in this case, the patient's head image needs to be synchronized with the brain image.

The guide device modeling module 230 may include a face mask modeling module 231, a stimulation apparatus holder modeling module 232, and a bridge modeling module 233.

The face mask modeling module 231 may generate the shape of the face mask 110 including the vertical guides 111b, 114, and 113b and the horizontal guides 111a, 112, and 113a based on the 3D image of the patient's face and information about the landmarks implemented by the face modeling module 210.

At least one of the first vertical guide 111b to the third vertical guide 113b may be included and the eye region may be deleted and unnecessary regions may be omitted while at least one of the second vertical guide 114 and the third vertical guide 113b is included to form openings 115 and 117. Strap connections 119 for connecting a band strap 150 may be added on the left and right sides and the upper side.

The stimulation apparatus holder 130 may be designed based on the shape of the brain stimulation apparatus 10 to be used for treating a patient, and may include the resting portion 131, the sidewall portion 133, the fixing protrusion 134, and the handle resting portion 135.

The bridge modeling module 233 may model a bridge 120 connecting the face mask 110 and the stimulation apparatus holder 130 when the position of the brain stimulation apparatus 10 determined by the procedure position determination module 250, i.e., the position of the stimulation apparatus holder 130, is spaced apart from the face mask 110.

The position and thickness of the bridge 120 may be designed such that one end of the bridge 120 is located at the face mask 110, the other end is located at the stimulation apparatus holder 130, and the stimulation apparatus holder 130 can be stably fixed through the bridge 120.

The bridge 120 may be configured as an integrated type, as shown in FIG. 8, or may be implemented as a separable type, as shown in FIG. 1. The bridge 120 design generated by the bridge modeling module 233 may be merged with the face mask 110 and the stimulation apparatus holder 130 generated by the face mask modeling module 231 to generate a model of the brain stimulation apparatus guide device 100.

For the separable bridge 120 as shown in FIG. 1, two models may be generated, one for the face mask 110 and the first bridge 121 and the other for the stimulation apparatus holder 130 and the second bridge 122.

When an existing manufactured design of the stimulation apparatus holder 130 is used, the model of the face mask 110 and the first bridge 121 may be combined with the model of the stimulation apparatus holder 130 and the second bridge 122 without generating a separate model, and the first bridge 121 may be designed such that the stimulation apparatus holder 130 is located at the procedure position. A model of the guide device forming module 260 in which the face mask 110 and the first bridge 121 are merged may be generated.

The guide device forming module 260 may be printed using a 3D printer based on the designed model of the brain stimulation apparatus guide device 100. In the case of an integrated type, the brain stimulation apparatus guide device 100 may be printed in one piece. In the case of a separable type, only a part of the brain stimulation apparatus guide device 100 in the form of the face mask 110 and the first bridge 121 combined may be printed.

FIG. 11 is a view showing a method of manufacturing a brain stimulation apparatus guide device 100 according to a further aspect of the present disclosure. The method of manufacturing the brain stimulation apparatus guide device 100 using the manufacturing apparatus of FIG. 10 will be described in detail.

Three-dimensional image information of a patient is received (S310). The three-dimensional image information may include a brain image captured using one of medical imaging devices, such as a magnetic resonance imaging (MRI) device, a functional MRI device, a computed tomography (CT) device, a positron emission tomography (PET) device, and an optical scanner.

The brain map generation module 220 generates a three-dimensional brain map of the patient based on the brain image of the patient. The brain map generation module identifies the region associated with the disease in the brain image, and compares the brain map of the patient with the brain of a normal person to generate a patient's brain map (first information) in which an abnormal point can be identified (S320).

Subsequently, the patient's brain map (first information) is compared with standardized brain stimulation target information (second information) that requires stimulation for treatment with the brain stimulation apparatus 10 to extract a target point (third information) to be stimulated with the brain stimulation apparatus 10 (S330 and S340).

The target point determination module 240 extracts a valid target point corresponding to the standardized stimulation point (second information) among the target points on the patient's brain map (first information) as a final target point (third information).

The point where the brain stimulation apparatus 10 is to be located to stimulate the target point, i.e., a procedure position, is determined (S350). At the procedure position, not only the brain map but also the head are three-dimensionally modeled using the 3D image of the patient (S360). Information about landmarks corresponding to the horizontal guides 111a, 112, and 113a, which can guide the face mask 110 to the left and right, and the vertical guides 111b, 114, and 113b, which can guide the face mask in the vertical direction, may be extracted from the three-dimensional model of the head. Additionally, information about a landmark corresponding to the vertical guide 111c may be extracted.

Once the three-dimensional model of the head and the procedure position are determined, the brain stimulation apparatus guide device 100 may be modeled (S380). The modeling of the brain stimulation apparatus guide device 100 may model a face mask 110 that covers the region including landmarks in detail. The brain stimulation apparatus resting portion 131 and the bridge 120 may be modeled based on the modeling of the face mask 110 and the procedure position.

The bridge 120 connecting the stimulation apparatus holder 130 and the face mask 110 is necessary if the procedure position is spaced apart from the face mask 110, and the overlap region between the stimulation apparatus holder 130 and the face mask 110 may be omitted if the procedure position overlaps the face mask 110.

If the bridge 120 is configured as a separable type, as in the embodiment of FIG. 1, the brain stimulation apparatus resting portion 131 need not be modeled separately, and the brain stimulation apparatus guide device 100 may be modeled with only the first bridge 121 coupled to the face mask 110.

The brain stimulation apparatus guide device 100 may be printed using a 3D printer based on the modeling of the completed brain stimulation apparatus guide device 100 to manufacture a patient-customized brain stimulation apparatus guide device 100 (S390).

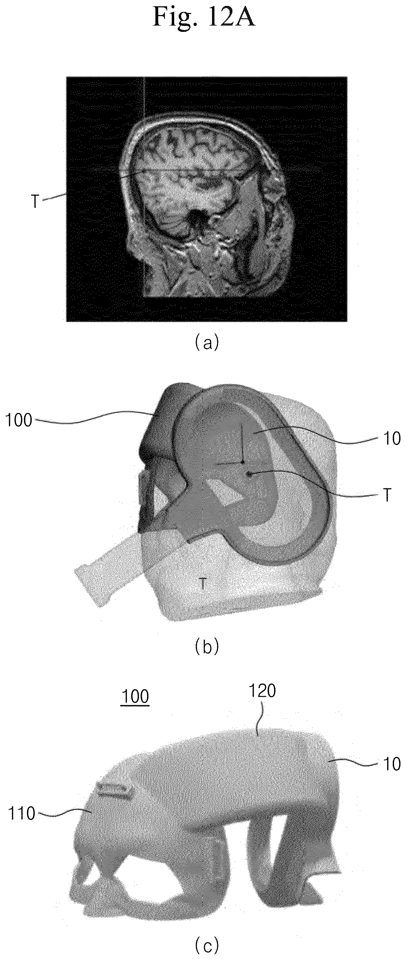

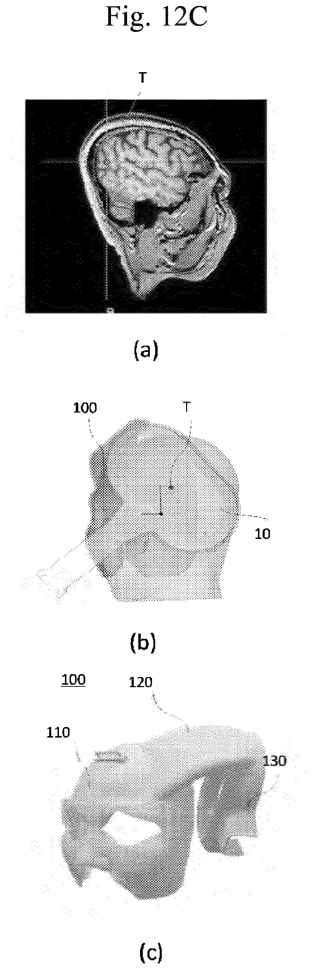

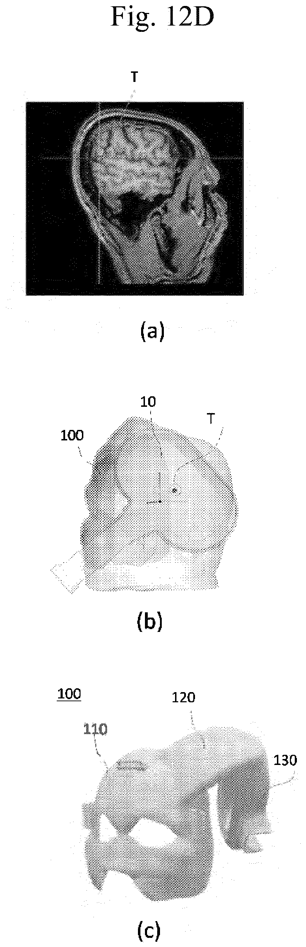

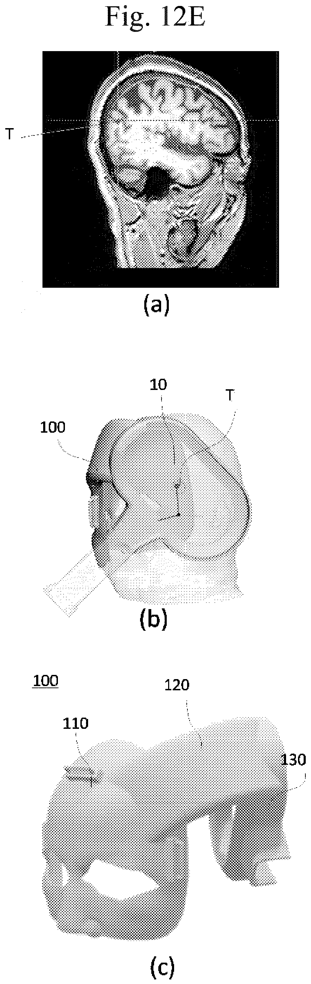

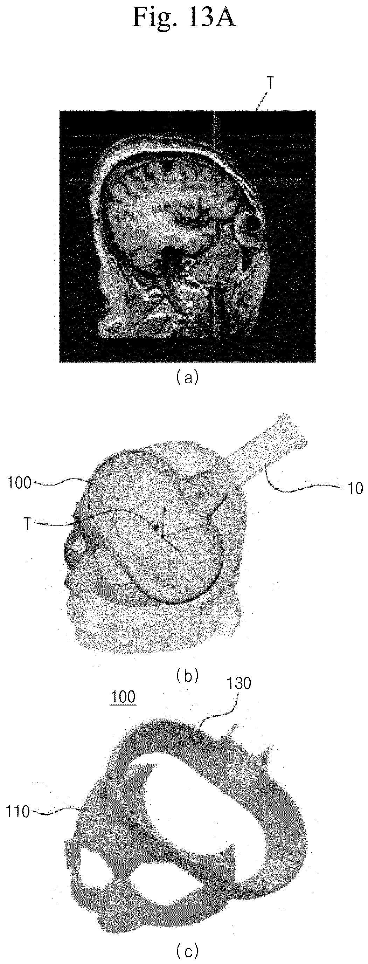

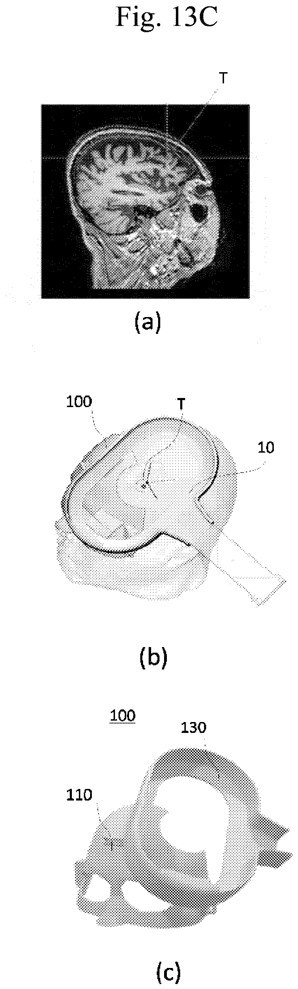

FIGs. 12A to 13C are views showing a manufacturing process according to the method of manufacturing the brain stimulation apparatus guide device 100 according to the further aspect of the present disclosure, wherein FIGs. 12A to 12E show an example of a method of manufacturing a brain stimulation apparatus guide device 100 for a patient with dementia and FIGs. 13A to 13C show an example of a method of manufacturing a brain stimulation apparatus guide device 100 for a patient with depression.

Referring to (a) of FIG. 12A, in the case of dementia, stimulating the angular gyrus or the left and right lateral parietal lobes has the effect of alleviating symptoms. The positions corresponding to the angular gyrus or the left and right lateral parietal lobes may be identified in the patient's brain image and determined to be a target point T.

The procedure position may be determined such that the target point is located such that the maximum point of the magnetic vector potential of the magnetic field of the brain stimulation apparatus 10 is located at the target point, as shown in (b) of FIG. 12A. Since the angular gyrus and the parietal lobe are located slightly posterior to the crown of the head, the procedure position may be located at an upper side of the occipital region to stimulate the angular gyrus and the parietal lobe. Since the position of the stimulation apparatus holder 130 is located on the occipital region side, a bridge 120 that connects the face mask 110 and the stimulation apparatus holder 130 is needed.

As shown in (c) of FIG. 12A, the brain stimulation apparatus guide device 100 including the face mask 110, the bridge 120, and the stimulation apparatus holder 130 may be modeled and output using a 3D printer.

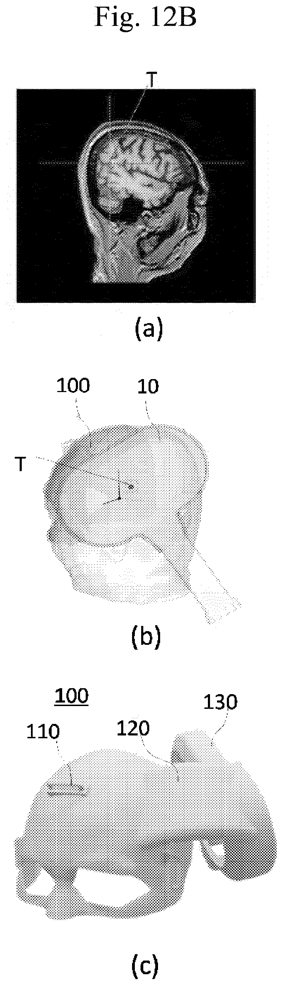

Other embodiments of the method of manufacturing the brain stimulation apparatus guide device 100 for the dementia patient according to the embodiment are further shown in FIGs. 12B to 12E.

Meanwhile, referring to (a) of FIG. 13A, the depression of a depression patient may be improved by stimulating the prefrontal region to increase the secretion of serotonin and dopamine. The prefrontal region in the patient's brain image may be determined as a target point T.

The procedure position may be determined such that the maximum point of magnetic vector potential of the magnetic field of the brain stimulation apparatus 10 is located at the target point, as shown in (b) of FIG. 13A. Since the prefrontal is located in close proximity to the forehead, the procedure position may be located on the forehead to stimulate the prefrontal. Since the position of the stimulation apparatus holder 130 overlaps the face mask 110, the bridge 120 is not required and a part of the overlap of the face mask 110 may be omitted.

As shown in (c) of FIG. 13A, the brain stimulation apparatus guide device 100 including the face mask 110 and the stimulation apparatus holder 130 may be modeled and output using a 3D printer.

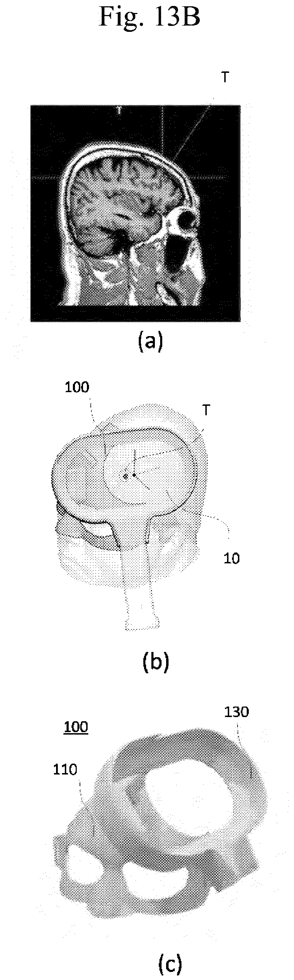

Other embodiments of the method of manufacturing the brain stimulation apparatus guide device 100 for the depression patient according to the embodiment are further shown in FIGs. 13B to 13C.

According to at least one embodiment of the present disclosure, the brain stimulation apparatus 10, which is a procedure tool, may be disposed at a correct position, thereby improving the effectiveness of the procedure.

The position does not change even if the patient moves, reducing the cumbersomeness of having to determine the position each time during repeated procedures.

While the embodiments of the present disclosure have been described above with reference to the accompanying drawings, it will be understood by one of ordinary skill in the art to which the present disclosure belongs that the present disclosure can be implemented in other specific forms without altering technical ideas or essential features thereof. Therefore, it should be understood that the embodiments described above are illustrative, not restrictive, in all respects.

## Claims

1. A brain stimulation apparatus guide device for positioning a brain stimulation apparatus for stimulating a patient's brain with an electric field or a magnetic field, the brain stimulation apparatus guide device comprising:
a face mask mounted to a face of the patient so as to cover at least a part of a nose, a temple, and a cheek of the patient; and
a stimulation apparatus holder fixed to the face mask, the stimulation apparatus holder being configured to allow a brain stimulation apparatus having a magnetic coil configured to generate a magnetic field mounted therein to rest thereon, wherein
the face mask comprises at least one of:
a first horizontal guide configured to wrap around left and right sides of a nasal bone of the patient;
a second horizontal guide located on left and right sides of the temple of the patient; and
a third horizontal guide located on the left and right sides of the cheek of the patient.

2. The brain stimulation apparatus guide device of claim 1, wherein the face mask comprises at least one of:
a first vertical guide located at an upper part of a nasal bone of the patient;
a second vertical guide configured to wrap around a brow bone of the patient; and
a third vertical guide configured to cover an upper part of the cheek of the patient.

3. The brain stimulation apparatus guide device of claim 2, wherein the face mask further comprises a second vertical guide configured to wrap around a part of a nose tip of the face.

4. The brain stimulation apparatus guide device of claim 1, wherein the brain stimulation apparatus comprises a transcranial magnetic stimulation (TMS) apparatus or a transcranial direct current stimulation (tDCS) apparatus.

5. The brain stimulation apparatus guide device of claim 1, wherein the stimulation apparatus holder comprises:
a resting face configured to wrap around a periphery of a front surface of the brain stimulation apparatus through which the magnetic field is radiated, the resting face comprising an opening formed in a center thereof;
a sidewall portion extending from the resting face, the sidewall portion being configured to wrap around a periphery of a side surface of the brain stimulation apparatus; and
a fixing protrusion extending from an end of the sidewall portion, the fixing protrusion being configured to allow a part of a rear surface of the brain stimulation apparatus to be hooked thereon.

6. The brain stimulation apparatus guide device of claim 5, wherein
the stimulation apparatus holder extends in one direction, and
the fixing protrusion is located on one side of the stimulation apparatus holder in a longitudinal direction.

7. The brain stimulation apparatus guide device of claim 5, further comprising a handle resting portion formed by omitting a part of the sidewall portion, the handle resting portion protruding in a lateral direction, the handle resting portion being configured to allow a handle of the brain stimulation apparatus to be located thereon.

8. The brain stimulation apparatus guide device of claim 1, wherein the face mask further comprises a band strap configured to fix the face mask to the face of the patient.

9. The brain stimulation apparatus guide device of claim 1, comprising:
a bridge configured to connect the face mask and the stimulation apparatus holder to each other, wherein
the bridge extends from an upper part of the face mask.

10. The brain stimulation apparatus guide device of claim 9, wherein the bridge comprises:
a first bridge connected to the face mask;
a second bridge connected to the stimulation apparatus holder; and
a fastener configured to fasten the first bridge and the second bridge to each other.

11. The brain stimulation apparatus guide device of claim 1, wherein
when a position of the stimulation apparatus holder and a position of the face mask at least partially overlap each other,
at least a part of an overlap region between the face mask and the stimulation apparatus holder is omitted.

12. The brain stimulation apparatus guide device of claim 8, wherein
the face mask comprises a strap loop configured to allow the band strap to be fixed thereto, and
the strap loop is provided in at least three on left and right sides and on an upper part of the face mask.

13. A method of manufacturing a brain stimulation apparatus guide device, the method comprising:
modeling a face mask configured to cover at least a part of a nose, a temple, and a cheek of a patient based on a three-dimensional image of a face of the patient;
a patient's brain map generation step of modeling a three-dimensional image of a brain of the patient;
extracting a target point based on the patient's brain map;
calculating a procedure position of a brain stimulation apparatus that generates a magnetic field such that a maximum point of a magnetic vector potential of the brain stimulation apparatus overlaps the target point;
modeling a first bridge extending from the face mask; and
a procedure guide forming step of outputting a procedure guide based on modeling of the face mask and modeling of the bridge, wherein
the first bridge comprises a fastening portion fastened to a second bridge extending from a ready-made stimulation apparatus holder to which the brain stimulation apparatus is mounted such that the stimulation apparatus holder is located at the procedure position.

14. The method of claim 13, wherein the step of modeling the face mask comprises at least one of:
modeling a first horizontal guide configured to wrap around left and right sides of a nasal bone of the face based on the three-dimensional image of the face of the patient;
modeling a second horizontal guide located on left and right sides of the temple of the face; and
modeling a third horizontal guide located on the left and right sides of the cheek of the face.

15. The method of claim 13, wherein the step of modeling the face mask comprises at least one of:
modeling a first vertical guide located at an upper part of a nasal bone of the face based on the three-dimensional image of the face of the patient;
modeling a second vertical guide configured to wrap around a brow bone of the face; and
modeling a third vertical guide configured to cover an upper part of the cheek of the face.

16. The method of claim 15, wherein the step of modeling the face mask further comprises modeling a fourth vertical guide configured to wrap around a part of a nose tip of the face.

17. The method of claim 13, wherein the step of extracting the target point comprises extracting a patient-customized target point to be stimulated with the brain stimulation apparatus based on the patient's brain map and standardized brain stimulation target information.

18. A method of manufacturing a brain stimulation apparatus guide device, the method comprising:
modeling a face mask configured to cover at least a part of a nose, a temple, and a cheek of a patient based on a three-dimensional image of a face of the patient;
a patient's brain map generation step of modeling a three-dimensional image of a brain of the patient;
extracting a target point from the patient's brain map;
calculating a procedure position of a brain stimulation apparatus that generates a magnetic field from a three-dimensional image of a head of the patient such that a maximum point of a magnetic vector potential of the brain stimulation apparatus overlaps the target point;
modeling a stimulation apparatus holder taking into account a shape of the brain stimulation apparatus and the procedure position; and
a brain stimulation apparatus guide device forming step of outputting a brain stimulation apparatus guide device based on modeling of the face mask and modeling of the stimulation apparatus holder.

19. The method of claim 15, wherein the step of modeling the face mask comprises at least one of:
modeling a first horizontal guide configured to wrap around left and right sides of a nasal bone of the face based on the three-dimensional image of the face of the patient;
modeling a second horizontal guide located on left and right sides of the temple of the face; and
modeling a third horizontal guide located on the left and right sides of the cheek of the face.

20. The method of claim 15, wherein the step of modeling the face mask comprises at least one of:
modeling a first vertical guide located at an upper part of a nasal bone of the face based on the three-dimensional image of the face of the patient;
modeling a second vertical guide configured to wrap around a brow bone of the face; and
modeling a third vertical guide configured to cover an upper part of the cheek of the face.

21. The method of claim 20, wherein the step of modeling the face mask further comprises modeling a fourth vertical guide configured to wrap around a part of a nose tip of the face.

22. The method of claim 18, further comprising:
modeling a bridge configured to connect the face mask and the stimulation apparatus holder to each other when the face mask and the stimulation apparatus holder are spaced apart from each other, wherein
the brain stimulation apparatus guide device forming step comprises outputting the brain stimulation apparatus guide device comprising the bridge.

23. The method of claim 18, wherein
when a position of the stimulation apparatus holder and a position of the face mask at least partially overlap each other,
the step of modeling the face mask comprises modeling the face mask with at least a part of the face mask that overlaps the stimulation apparatus holder omitted.

24. The method of claim 18, wherein the step of extracting the target point comprises extracting a patient-customized target point to be stimulated with the brain stimulation apparatus based on the patient's brain map and standardized brain stimulation target information.

25. An apparatus for manufacturing a brain stimulation apparatus guide device, the apparatus comprising:
a face modeling module configured to generate a three-dimensional image of a face of a patient based on an image of a head of the patient;
a brain map generation module configured to model a three-dimensional image of a brain of the patient based on an MRI image of the brain of the patient;
a target point determination module configured to extract a target point from the generated brain map;
a procedure position determination module configured to determine a procedure position of a brain stimulation apparatus that generates a magnetic field such that a maximum point of a magnetic vector potential of the brain stimulation apparatus is located at the target point;
a guide device modeling module configured to model a face mask configured to cover at least a part of a nose, a temple, and a cheek of the patient and a stimulation apparatus holder configured to mount the brain stimulation apparatus at the procedure position based on the three-dimensional image of the face of the patient; and
a guide device forming module configured to output a brain stimulation apparatus guide device based on modeling of the face mask and the stimulation apparatus holder.

26. The apparatus of claim 25, wherein
the guide device modeling module models a bridge configured to connect the face mask and the stimulation apparatus holder to each other when the face mask and the stimulation apparatus holder are spaced apart from each other, and
the guide device forming module outputs the brain stimulation apparatus guide device comprising the bridge.
